# EUROPEAN PATENT SPECIFICATION

(11) **EP 2 073 774 B1**
(45) Date of publication and mention of the grant of the patent: **17.12.2014**
(21) Application number: 07794178.9
(22) Date of filing: 23.08.2007
(51) Int. Cl.: A61F 13/20, A61F 13/551, B65D 30/10, B65F 1/00

(54) **DISPOSAL BAG FOR HYGIENE PRODUCTS AND METHOD OF MANUFACTURING THE SAME**
MÜLLBEUTEL FÜR HYGIENEPRODUKTE UND HERSTELLUNGSVERFAHREN DAFÜR
SAC JETABLE DESTINÉ À DES PRODUITS D'HYGIÈNE ET PROCÉDÉ DE FABRICATION CORRESPONDANT

(30) Priority: 01.09.2006 SE 0601803
(43) Date of publication of application: 01.07.2009
(73) Proprietor: Sanett AB, 126 32 Hägersten (SE)
(72) Inventor: ENLUND, Lina, 126 32 Hägersten (SE)
(74) Representative: Norris, Timothy Sweyn
(86) International application number: PCT/SE2007/050568
(87) International publication number: WO 2008/026994

(56) References cited:
- CN-Y- 201 073 444
- FR-A1- 2 620 684
- GB-A- 2 386 108
- JP-A- 2003 180 740
- JP-A- 2004 097 251
- US-A- 6 001 434
- US-A- 6 102 457
- US-A1- 2002 178 482

## Description

The present invention relates to a disposal bag for hygiene products according to the preamble of the accompanying claim 1. It also relates to a method developed when manucturing such a disposal bag of the type which is disclosed in the preamble to claim 5. It relates as well to a method developed for manufacturing such a disposal bag of the type disclosed in claim 6.

### Background of the invention

In order to prevent plugged toilets, ladies rooms are usually provided with a supply of bags, in which sanitary napkins and tampons can be enclosed and discarded into a container intended for this purpose. With regard to tampons, however, many women have continued to allow the extracted tampon to drop down into the toilet and be flushed away, sometimes causing significant problems in the sewage system. Smaller bags intended for tampons, which could be used to receive the tampon directly upon extraction with a string, have been suggested e.g. in Japanese Patent Specification No. 2004097251 (to KAO Corporation) and French Patent Specification 2620684. These solutions are, however, complicated in their construction, requiring a number of different steps to manufacture, and use tape or a string for closure. Other solutions which are described e.g. in US Patent Specification 6,402,727 B1 or Japanese Patent Specification 2003180740, comprise a napkin or a sheet of material which is intended to be folded around and enclose the used tampon. These are, however, more difficult to use and require tape or a string to close.

### The purpose of the present invention

The purpose of the present invention is to achieve a disposal bag which is easy to use for immediate reception of tampons and which is easy to close, manufacture and store before use, with an absolute minimum of material and parts. It is also a purpose of the present invention to achieve a method of rapid, simple and economical manufacture of such bags, which can be supplied in rolls ready for use or subsequent packing steps.

These and other purposes are achieved with a disposal bag of the type described by way of introduction which has the characteristics disclosed in claim 1, with a method of the type described by way of introduction which has the characteristics disclosed in claim 5 and with a method of the type described by way of introduction which has the characteristics disclosed in claim 6. Advantageous embodiments are disclosed in the subclaims.

The disposal bag which is impermeable to liquid and odours is very simple to hold open in one hand to receive the tampon when the woman extracts it from her vagina, which prevents unpleasantness and the risk of getting menstrual blood or liquid on clothes etc.

### Figures

The present invention will be described below with reference to the accompanying drawings of which:
Fig. 1 shows a disposal bag according to the invention,
Fig. 2 (a-d) shows four steps in the use of the disposal bag according to the invention when receiving a tampon,
Fig. 3 shows how the bags may be welded and cut from a double web of thin plastic material in a first manufacturing process,
Fig. 4 schematically shows how the various components in a manufacturing process according to this first manufacturing method could be arranged.

### Detailed description of preferred embodiments

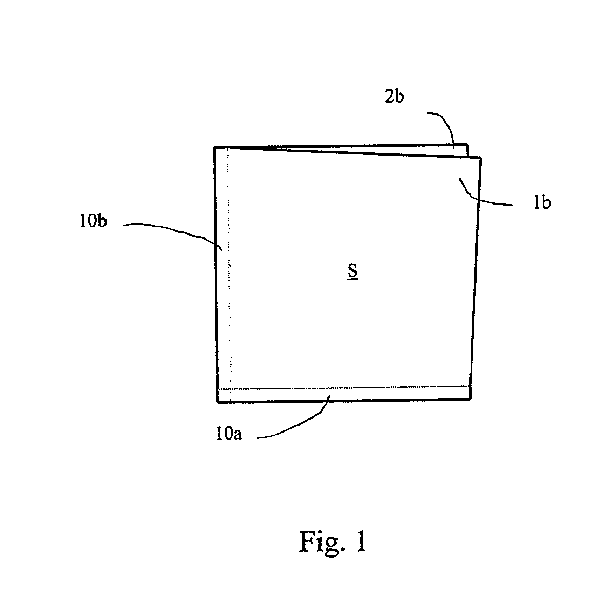

Fig. 1 shows a disposal bag S which consists of a simple square sheet of plastic 1 b with a similar square sheet of plastic 2b on top of it. These two sheets are welded together or glued together on only two of their edges 10a and 10b to form the disposal bag S.

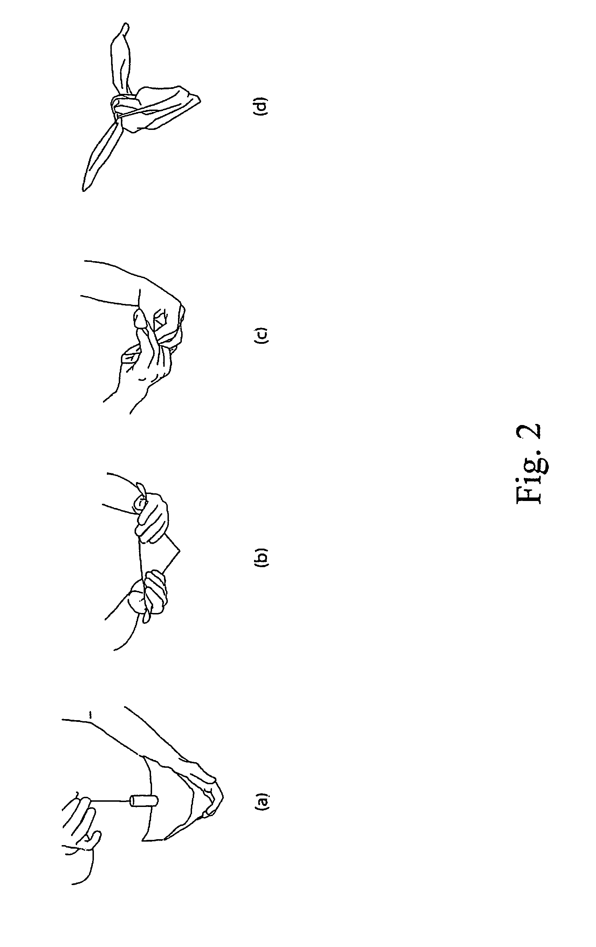

The plastic sheet, or any other material which is impermeable to liquid and odour is sufficiently rigid to keep its open shape when receiving the tampon (Fig. 2(a)) but still sufficiently flexible so that the free corners can be tied together as shown in Figs. 2 (b)-(c)). Fig. 2(d) shows the resulting package which can be placed in a refuse container or even be placed in a pocket or handbag if it is not possible to dispose of the package directly.

When extracting a used tampon the user is many times unwilling to lift it above the toilet bowl for fear of spoiling clothing or of mere displeasure. In order to rapidly get rid of a used tampon when extracted, the user has often immediately dropped the tampon down into the toilet and flushed it away with the disadvantages to plumbing and sewage treatment which this involves. The wide open bag according to the invention can be held in one hand next to the vagina upon reception of the tampon so that the user never needs to lift up the unprotected tampon.

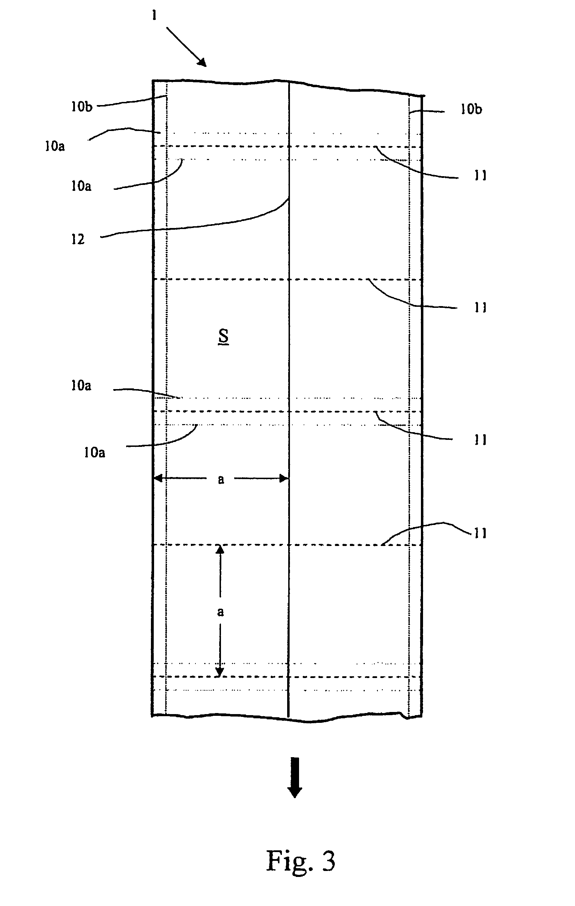

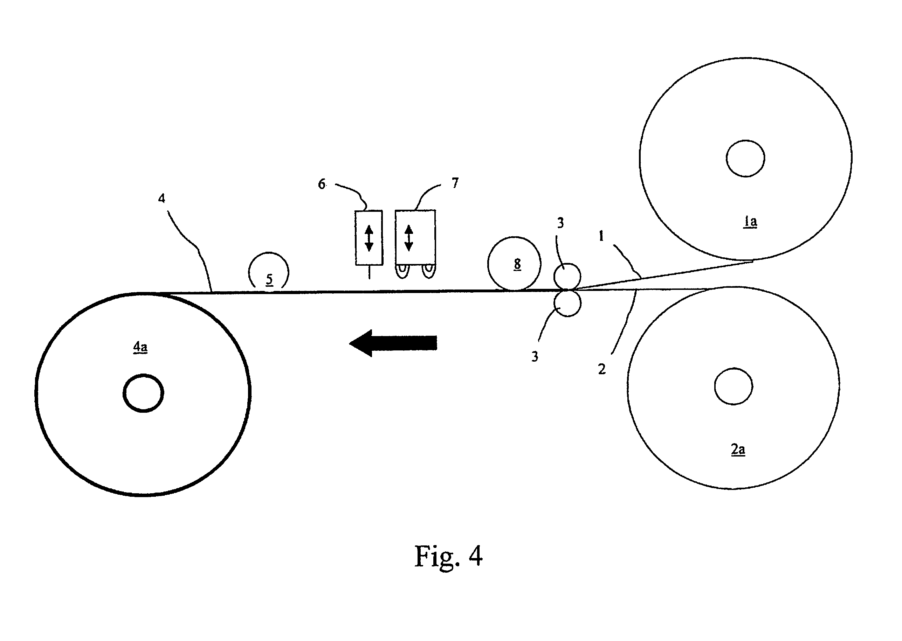

The disposal bag according to the invention is surprisingly simple and economical to manufacture by means of the methods according to the invention. The methods make it possible to deliver bags on rolls with tear-off perforations between the bags for use in this form or for further processing and packaging. Figs. 3 and 4 show how a double sheet web can be welded and cut according to the invention. The components in Fig. 4, however, are only shown symbolically and the components can be arranged in another order within the scope of the invention. The double sheet web consists, according to a first embodiment, of two identical sheets 1 and 2, of width 2a, which run from two different rolls 1a and 2a. The two different webs 1 and 2 are brought together by two rollers 3, whereafter two welding rollers 8 provide two longitudinal welding lines 10b along either side of the sheeting web. The welding rollers 8 are only shown schematically here and have of course counter rolls or support plates on the other side of the web which are not shown. Glueing, laser welding or ultra-sonic welding can of course replace the schematically shown welding wheels 8. A pair of transverse weld lines 10a are made at even spacing "2 x a" as the web is advanced. These welding components are shown schematically as component 7, which is lowered intermittently at the correct moment. This component can of course also be replaced by other welding means such as laser welding or ultra-sonic welding means. A transverse perforation instrument 6 extends transversely across the web and produces perforation lines 11 at even spacing "a" as shown in Fig. 3. Thereafter, the cutting disc 5 cuts the web into two parts and two webs of bags are rolled up on two rolls 4a.

According to a second method of manufacture according to the invention, the double layer of plastic sheeting in the web is achieved by folding in the middle the plastic sheeting longitudinally when rolled off from a roll of width 4 x a. This will make one of the longitudinal welds 10b in the first method described above unnecessary.

It is also possible of course to cut the web in both methods instead of making perforations 11 and thereafter collecting the individual bags. The simple and thin form of the bags makes it possible to include a suitable numer of disposal bags in the cover of a package of tampons.

## Claims

1. A disposal bag intended for receiving a used absorbant hygiene article, such as a tampon, wherein the disposal bag comprises a liquid impermeable material, and an opening intended for insertion of the used absorbent hygiene article, and wherein the bag consists of two essentially square sheets (1 b, 2b), lying on top of each other and made of an at least relatively liquid impermeable material,
**characterized in that**
the two essentially square sheets are bonded to each other along only two of the square sides lying adjacent to each other, said sheets being of such dimensions that the bag permits reception of the hygiene article and subsequent manual tying of the two free corners of the sheets for closing the bag, and
the disposal bag is joined by tear-off perforations to at least one adjacent similar bag on a roll, prior to use.

2. A disposal bag according to claim 1, **characterized in that** the two sheets lying on top of each other are bonded to each other along one of their square sides, one material sheet being made in one piece with the other which is folded over said one sheet.

3. A disposal bag according to claim 1 or 2, **characterized in that** one or both of the bonds are achieved by welding.

4. A disposal bag according to claim 1 or 2, **characterized in that** one or both of the bonds are achieved by gluing.

5. A method of manufacturing a disposal bag according to claim 1, **characterized in that** the method includes:
(a) advancing a web (2) of liquid impermeable material having a width which is two times a width a of the disposal bag;
(b) with another identical web (1) on top thereof, forming a single double sheet web;
(c) continuously welding together (10b) along the two sides of the double sheet web;
(d) intermittently double bead transversally welding (10a) at intervals 2a in an advancing direction of the double sheet web;
(e) continuously mid-cutting (12) the web in the advancing direction of the web; and
(f) subsequently intermittently cross-cutting or perforating (11) the double sheet web at each interval *a* in the advancing direction of the web, with every other cross-cut or perforation being in a middle of each double-bead transverse weld (10a).

6. A method of manufacturing a disposal bag according to claim 1, **characterized in that** the method includes:
(a) double folding in a longitudinal direction of a web of liquid impermeable material of unfolded width *4a,* i.e. four times a width *a* of the finished disposal bag;
(b) advancing the double folded web of liquid impermeable material, wherein the double folded web has a width which is two times the width *a* of the disposal bag;
(c) continuously welding together along an edge of the double folded web which is opposite to a folded edge of the double folded web;
(d) intermittently double-beaded transverse welding at intervals *2a* in the advancing direction of the web;
(e) continuously mid-cutting the web in the advancing direction of the web; and
(f) subsequently intermittently cross-cutting or perforating the web at each interval *a* in the advancing direction of the web with every other cross-cutting or perforation in a middle of each double-beaded transverse welding.

## Patentansprüche

1. Wegwerfbeutel, der für die Aufnahme eines benutzten absorbierenden Hygieneartikels, wie beispielsweise eines Tampons, vorgesehen ist, wobei der Wegwerfbeutel ein flüssigkeitsundurchlässiges Material und eine Öffnung umfasst, die für das Einführen des benutzten absorbierenden Hygieneartikels vorgesehen ist, und wobei der Beutel aus zwei im Wesentlichen quadratischen Bögen (1b, 2b) besteht, die übereinander liegen und aus einem zumindest relativ flüssigkeitsundurchlässigen Material bestehen, **dadurch gekennzeichnet, dass**
die zwei im Wesentlichen quadratischen Bögen an nur zwei nebeneinanderliegenden Seiten des Quadrats aneinander gebunden sind, wobei die Bögen derart bemessen sind, dass der Beutel die Aufnahme des Hygieneartikels und das nachfolgende manuelle Verknoten der zwei freien Ecken der Bögen zum Verschließen des Beutels ermöglicht, und
der Wegwerfbeutel vor dem Gebrauch auf einer Rolle mittels Abreißperforationen mit mindestens einem angrenzenden ähnlichen Beutel verbunden ist.

2. Wegwerfbeutel nach Anspruch 1, **dadurch gekennzeichnet, dass** die zwei übereinanderliegenden Bögen an einer ihrer Quadratseiten aneinander gebunden sind, wobei ein Materialbogen mit dem anderen in einem Stück gefertigt ist, wobei ein Bogen über den anderen gefaltet ist.

3. Wegwerfbeutel nach Anspruch 1 oder 2, **dadurch gekennzeichnet, dass** eine der Bindungen oder beide durch Schweißen erzielt werden.

4. Wegwerfbeutel nach Anspruch 1 oder 2, **dadurch gekennzeichnet, dass** eine der Bindungen oder beide durch Kleben erzielt werden.

5. Verfahren zur Herstellung eines Wegwerfbeutels nach Anspruch 1, **dadurch gekennzeichnet, dass** das Verfahren Folgendes beinhaltet:
(a) Vorrücken einer Bahn (2) aus flüssigkeitsundurchlässigem Material mit einer Breite, die das Zweifache einer Breite *a* des Wegwerfbeutels beträgt;
(b) Bilden einer einzigen Doppelbogenbahn mit einer anderen, identischen Bahn (1) auf deren Oberseite;
(c) kontinuierliches Aneinanderschweißen (10b) entlang der zwei Seiten der Doppelbogenbahn;
(d) absatzweises Doppelnahtschweißen in Querrichtung (10a) in Intervallen *2a* in einer Vorrückrichtung der Doppelbogenbahn;
(e) kontinuierliches Schneiden (12) der Bahn in der Mitte in Vorrückrichtung der Bahn; und
(f) nachfolgendes absatzweises Querschneiden oder Perforieren (11) der Doppelbogenbahn in jedem Intervall *a* in der Vorrückrichtung der Bahn, wobei jeder zweite Querschnitt oder jede zweite Perforation in einer Mitte jeder querliegenden Doppelschweißnaht (10a) liegt.

6. Verfahren zur Herstellung eines Wegwerfbeutels nach Anspruch 1, **dadurch gekennzeichnet, dass** das Verfahren Folgendes beinhaltet:
(a) Doppelfalten einer Bahn aus flüssigkeitsundurchlässigem Material mit einer nicht gefalteten Breite *4a*, d. h. vier Mal eine Breite *a* des fertigen Wegwerfbeutels, in eine Längsrichtung;
(b) Vorrücken der doppelt gefalteten Bahn aus flüssigkeitsundurchlässigem Material, wobei die doppelt gefaltete Bahn eine Breite aufweist, die das Zweifache der Breite *a* des Wegwerfbeutels beträgt;
(c) kontinuierliches Aneinanderschweißen entlang einer Kante der doppelt gefalteten Bahn, die einer gefalteten Kante der doppelt gefalteten Bahn gegenüberliegt;
(d) absatzweises Doppelnahtschweißen in Querrichtung in Intervallen *2a* in einer Vorrückrichtung der Doppelbogenbahn;
(e) kontinuierliches Schneiden der Bahn in der Mitte in Vorrückrichtung der Bahn; und
(f) nachfolgendes absatzweises Querschneiden oder Perforieren der Bahn in jedem Intervall *a* in der Vorrückrichtung der Bahn, wobei jeder zweite Querschnitt oder jede zweite Perforation in einer Mitte jeder querliegenden Doppelschweißnaht liegt.

## Revendications

1. Sac à ordures destiné à recevoir un article d'hygiène absorbant usagé, tel qu'un tampon hygiénique, dans lequel le sac à ordures comprend un matériau imperméable aux liquides, et une ouverture destinée à l'insertion de l'article d'hygiène absorbant usagé, et dans lequel le sac est constitué de deux feuilles essentiellement carrées (1 b, 2b), placées l'une sur l'autre et faites d'un matériau au moins relativement imperméable aux liquides,
**caractérisé en ce que**
les deux feuilles essentiellement carrées sont liées l'une à l'autre seulement de deux des côtés carrés placés adjacents l'un à l'autre, lesdites feuilles ayant des dimensions telles que le sac permette la réception de l'article d'hygiène et l'attachement manuel subséquent des deux coins libres des feuilles pour fermer le sac, et
le sac à ordures est solidarisé par des perforations à déchirer à au moins un sac similaire adjacent sur un rouleau, avant d'être utilisé.

2. Sac à ordures selon la revendication 1, **caractérisé en ce que** les deux feuilles placées l'une sur l'autre sont liées l'une à l'autre le long de l'un de leurs côtés carrés, une feuille de matériau étant créée d'une pièce avec l'autre qui est pliée au-dessus de ladite une feuille.

3. Sac à ordures selon la revendication 1 ou 2, **caractérisé en ce que** l'une ou les deux liaisons sont réalisées par soudage.

4. Sac à ordures selon la revendication 1 ou 2, **caractérisé en ce que** l'une ou les deux liaisons sont réalisées par collage.

5. Méthode de fabrication d'un sac à ordures selon la revendication 1, **caractérisée en ce que** la méthode comprend :
(a) l'avancée d'une toile (2) d'un matériau imperméable aux liquides ayant une largeur qui est le double d'une largeur a du sac à ordures ;
(b) avec une autre toile identique (1) au-dessus d'elle, la formation d'une unique toile à double feuille ;
(c) le soudage mutuel continu (10b) le long des deux côtés de la toile à double feuille ;
(d) le soudage transversal à double bourrelet par intermittence (10a) à des intervalles 2a dans une direction d'avancée de la toile à double feuille ;
(e) la découpe médiane en continu (12) de la toile dans la direction d'avancée de la toile ; et
(f) consécutivement et par intermittence, la découpe transversale ou la perforation (11) de la toile à double feuille à chaque intervalle a dans la direction d'avancée de la toile, avec une découpe transversale ou perforation sur deux réalisée au milieu de chaque soudure transversale à double bourrelet (10a).

6. Méthode de fabrication d'un sac à ordures selon la revendication 1, **caractérisée en ce que** la méthode comprend :
(a) le double pliage dans une direction longitudinale d'une toile en matériau imperméable aux liquides d'une largeur dépliée 4a, c'est-à-dire le quadruple d'une largeur a du sac à ordures fini ;
(b) l'avancée de la toile doublement pliée en matériau imperméable aux liquides, dans laquelle la toile doublement pliée a une largeur qui est le double de la largeur a du sac à ordures ;
(c) le soudage mutuel continu le long d'un bord de la toile doublement pliée qui est à l'opposé d'un bord plié de la toile doublement pliée ;
(d) le soudage transversal à double bourrelet par intermittence à des intervalles 2a dans la direction d'avancée de la toile ;
(e) la découpe médiane continu de la toile dans la direction d'avancée de la toile ; et
(f) consécutivement et par intermittence, la découpe transversale ou la perforation de la toile à chaque intervalle a dans la direction d'avancée de la toile avec une découpe transversale ou perforation sur deux réalisée au milieu de chaque soudure transversale à double bourrelet.
